# EUROPEAN PATENT SPECIFICATION

(11) **EP 3 530 670 B1**
(45) Date of publication and mention of the grant of the patent: **28.02.2024**
(21) Application number: 17862926.7
(22) Date of filing: 18.10.2017
(51) Int. Cl.: C07K 14/435, G01N 33/579, C12N 15/09

(54) **METHOD FOR PRODUCING ENDOTOXIN DETECTING AGENT COMPRISING RECOMBINANT LIMULUS FACTOR C AND USE THEREOF**
VERFAHREN ZUR HERSTELLUNG EINES REKOMBINANTEN LIMULUS-FAKTOR C ENTHALTENDEN ENDOTOXIN-NACHWEISMITTELS UND DESSEN VERWENDUNG
MÉTHODE DE PRODUCTION D'UN AGENT DÉTECTEUR D'ENDOTOXINE COMPRENANT DU FACTEUR C DE LIMULUS RECOMBINANT ET SON UTILISATION

(30) Priority: 18.10.2016 JP 2016204729
(43) Date of publication of application: 28.08.2019
(60) Divisional application: 24150705.2
(73) Proprietor: Seikagaku Corporation, Tokyo 100-0005 (JP)
(72) Inventor: MIZUMURA, Hikaru, Tokyo 100-0005 (JP); KOBAYASHI, Yuki, Tokyo 100-0005 (JP); ODA, Toshio, Tokyo 100-0005 (JP)
(74) Representative: Müller-Boré & Partner Patentanwälte PartG mbB
(86) International application number: PCT/JP2017/037629
(87) International publication number: WO 2018/074498

(56) References cited:
- EP-A1- 2 930 241
- EP-A1- 3 591 049
- WO-A1-2014/092079
- JP-A- 2014 510 898
- JP-A- 2015 141 088
- JP-A- 2015 536 158
- FUMINORI TOKUNAGA ET AL: "Further Studies on Lipopolysaccharide-Sensitive Serine Protease Zymogen (Factor C): Its Isolation from Limulus polyphemus Hemocytes and Identification as an Intracellular Zymogen Activated by [alpha]-Chymotrypsin, Not by Trypsin1", JOURNAL OF BIOCHEMISTRY, vol. 109, no. 1, 1 January 1991 (1991-01-01), pages 150-157, XP055682343, GB ISSN: 0021-924X, DOI: 10.1093/oxfordjournals.jbchem.a123337
- DING JEAK LING ET AL: "Chapter 9: Endotoxin Detection - from Limulus Amebocyte Lysate to Recombinant Factor C", 1 January 2010 (2010-01-01), ENDOTOXINS: STRUCTURE, FUNCTION AND RECOGNITION - SERIES : SUBCELLULAR BIOCHEMISTRY IN: SUBCELLULAR BIOCHEMISTRY : REVIEWS AND ESSAYS DEALING WITH THE FUNCTION, GENETICS, BIOGENESIS AND EVOLUTION OF SUB-CELLULAR COMPONENTS, ISSN 0096-8757 ; VOL. 53;, XP009510157, ISBN: 978-90-481-9077-5 * the whole document *
- NAKAMURA S ET AL: "Fractionation of Limulus amebocyte lysate - Characterization of activation of the proclotting enzyme by an endotoxin-mediated activator", BIOCHIMICA ET BIOPHYSICA ACTA. PROTEIN STRUCTURE AND MOLECULAR ENZYMOLOGY, ELSEVIER, AMSTERDAM; NL, vol. 707, no. 2, 5 October 1982 (1982-10-05), pages 217-225, XP023476507, ISSN: 0167-4838, DOI: 10.1016/0167-4838(82)90354-5 [retrieved on 1982-10-05]

## Description

The present invention relates to specific endotoxin detecting agents, as well as respective methods for detecting an endotoxin using said agents.

Horseshoe crabs are called "living fossils" and there exist only two genera and four species on Earth. As the genera, there are only "the genus Limulus" living only in the east coast of the North American Continent and "the genus Tachypleus" living only in the southeast sea area in Asia.

A distribution in which one organism group is separately distributed in regions far from each other is called "discontinuous distribution", and organisms which are separated in two regions on Earth like horseshoe crabs are very rare.

The organisms belonging to the genus Limulus are assigned to only one species: Limulus polyphemus, and the organisms belonging to the genus Tachypleus are assigned to only three species: Tachypleus tridentatus, Tachypleus gigas, and Tachypleus rotundicauda (also called Carcinoscorpius rotundicauda) .

It is known that when an extract of amoebocytes (amoebocyte lysate) present in the blood of this organism comes into contact with an endotoxin, the extract clots. By utilizing this property, the amoebocyte lysate has been widely used for the quality control of pharmaceutical preparations or the like as a reagent for detecting an endotoxin with high sensitivity.

This reagent is called "lysate reagent". Further, the property of causing clotting by an endotoxin was found in Limulus polyphemus for the first time, and therefore, this reagent is sometimes called "Limulus reagent" or "LAL (Limulus amoebocyte lysate) reagent". Even a reagent using an amoebocyte lysate derived from the genus Tachypleus is customarily called "Limulus reagent" or "LAL reagent".

At present, the lysate reagent (Limulus reagent or LAL reagent) used by pharmaceutical manufacturers in Japan, U.S. and Europe is derived from Limulus polyphemus as a raw material. The number of captures of Limulus polyphemus is strictly limited due to extinction concerns, however, some reports also say that the population is still decreasing.

In order to protect horseshoe crabs, there has been an idea that the reagent is prepared by artificially producing proteins in the amoebocyte lysate using a recombinant technique (PTL 1 to PTL 8). Then, by utilizing recombinant proteins, products such as PyroGene (trademark) (Lonza), EndoZyme (trademark) (Hyglos), and PyroSmart (trademark) (Seikagaku Corporation) have been actually launched in the market.

However, any of these uses recombinant proteins derived from an organism of the genus Tachypleus, and there has been no report of a reagent using recombinant proteins derived from Limulus polyphemus which has been widely spread as the lysate reagent.

The reason for this is because proteins involved in a mechanism of occurrence of clotting by an endotoxin and genes thereof in Limulus polyphemus have not been completely identified structurally and functionally.

In the genus Tachypleus, for example, with respect to factor C which is one of the proteins involved in the clotting mechanism, a full gene sequence and a full amino acid sequence derived from Tachypleus tridentatus have been reported in 1991 (NPL 1), and a full gene sequence and a full amino acid sequence derived from Tachypleus rotundicauda (Carcinoscorpius rotundicauda) have been reported in 1995 (NPL 2).

However, in Limulus polyphemus, complete identification of these proteins and genes from both structural and functional aspects has not been achieved even though 20 years or more has passed since the reports regarding the genus Tachypleus described above were published. This is because accurate determination of the full-length sequence structures and elucidation of the expression and functions of these proteins and genes could not be achieved by general techniques due to various differences in biomolecules, diversities, etc. because of differences in genera or species of horseshoe crabs.

In fact, as part of the invertebrate genome project using a next-generation sequencer, a comprehensive analysis of genes of Limulus polyphemus has been carried out (http://genome.wustl.edu/genomes/detail/limulus-polyphemus /), however, the full-length sequences of all proteins and genes involved in the clotting mechanism of Limulus polyphemus have not been elucidated yet. This fact also supports the difficulty in accurate determination of the full-length sequence structures of these proteins and genes in Limulus polyphemus.

In this context, Tokunaga et al. (Tokunaga, F. et al.; J. Biochem., 109; pp. 150-157; 1991) describes the isolation of Factor C from Limulus polyphemus and its characterization as an intracellular zymogen that is activated by (α-chymotrypsin. Further, EP 2 930 241 A1 describes the expression of a recombinant horseshoe crab Factor C in mammalian cells.

### Patent Literature

PTL 1: US Patent No. 5,712,144
PTL 2: US Patent No. 5,716,834
PTL 3: US Patent No. 5,858,706
PTL 4: US Patent No. 5,985,590
PTL 5: US Patent No. 6,645,724
PTL 6: WO 2008/004674
PTL 7: JP-T-2014-510898
PTL 8: WO 2014/092079

### Non Patent Literature

NPL 1: Muta T et al., Journal of Biological Chemistry, 266, 6554-6561 (1991)
NPL 2: Ding JL et al., Molecular Marine Biology and Biotechnology, 4(1), 90-103 (1995)

An object of the present invention is to provide all full-length recombinant proteins involved in the clotting mechanism of Limulus polyphemus, cDNAs encoding the same, and applications thereof.

The present inventors were successful in the acquisition of full-length cDNAs encoding all protein factors involved in the clotting mechanism of Limulus polyphemus which no one had ever been successful for a long time, and the expression of all recombinant proteins thereof, and further found that these can be utilized in various applications, and thus completed the present invention.

That is, the present invention is characterized in the appended claims.

The figures show:
[FIG. 1] FIG. 1 is an electrophoresis photograph of a PCR product in a process for acquiring a cDNA of a protein having factor C activity of Limulus polyphemus.
[FIG. 2] FIG. 2 is an electrophoresis photograph of a PCR product in a process for acquiring a cDNA of a protein having factor C activity of Limulus polyphemus.
[FIG. 3] FIG. 3 is a figure showing the activity of cleaving a synthetic substrate by contact of each recombinant protein alone or various mixtures of recombinant proteins with an endotoxin.
[FIG. 4] FIG. 4 is a figure showing a calibration curve of an endotoxin using a mixture of three types of recombinant proteins.

### <1> Recombinant First Protein and Production Method Therefor

### (1) Recombinant First Protein

A recombinant first protein is a recombinant protein having factor C activity.

The recombinant first protein is specifically a recombinant protein which is either of the following:
(I) a recombinant protein that contains an amino acid sequence of the protein of Limulus polyphemus having factor C activity; and
(II) a recombinant protein that is a variant of the above (I) and has factor C activity.

Examples of the amino acid sequence of the protein of Limulus polyphemus having factor C activity include the amino acid sequence represented by SEQ ID NO: 2 or 4. Further, examples of a base sequence encoding such an amino acid sequence include the base sequence represented by SEQ ID NO: 1 or 3.

The recombinant first protein may be more specifically a recombinant protein which is any of the following:
(A) a recombinant protein that contains the amino acid sequence represented by SEQ ID NO: 2 or 4;
(B) a recombinant protein that contains an amino acid sequence having a 95% or more identity with the amino acid sequence represented by SEQ ID NO: 2 or 4, and has factor C activity;
(C) a recombinant protein that is encoded by a DNA containing a base sequence having a 95% or more identity with the base sequence represented by SEQ ID NO: 1 or 3, and has factor C activity; and
(D) a recombinant protein that is encoded by a DNA hybridizing to a DNA composed of a complementary sequence to the base sequence represented by SEQ ID NO: 1 or 3 under stringent conditions, and has factor C activity.

Note that the expression of "containing an amino acid sequence" or "containing a base sequence" also includes a case of "being composed of the amino acid sequence" or a case of "being composed of the base sequence".

This protein was acquired for the first time in the world by the method disclosed in Examples of this description, and the full-length sequence structure and function thereof were elucidated for the first time. The function of the protein is a function of turning into an active form in the presence of an endotoxin and converting a protein having factor B activity such as the below-mentioned second protein into an active form (referred to as "factor C activity" in this application document) . The recombinant first protein may be a protein that shows the factor C activity in combination with at least the second protein such as a recombinant second protein.

Whether having the factor C activity can be confirmed by detecting the progress of a cascade reaction in the presence of an endotoxin when combining a target recombinant protein with a protein (which is not in an active form, hereinafter referred to as "inactive form") having factor B activity such as the second protein (inactive form), a protein (inactive form) having proclotting enzyme activity such as a third protein (inactive form), and a substrate for detection.

The "cascade reaction" refers to a series of reactions in which a protein (inactive form) having factor C activity is activated by an endotoxin, a protein (inactive form) having factor B activity is activated by the protein (active form) having factor C activity, and a protein (inactive form) having proclotting enzyme activity is activated by the protein (active form) having factor B activity. The progress of the cascade reaction can be detected by cleavage of a substrate for detection.

Among the recombinant first proteins, the most preferred is the above-mentioned recombinant protein (I) such as the above-mentioned recombinant protein (A), but the recombinant protein may be a variant thereof as long as it has factor C activity.

The variant may be, for example, a recombinant protein that contains an amino acid sequence including a substitution, a deletion, an insertion, and/or an addition of one or several amino acids at one or several sites in an amino acid sequence (for example, the amino acid sequence represented by SEQ ID NO: 2 or 4) of the recombinant first protein as described above, and has factor C activity. The term "one or several" may be, for example, 1 to 20, 1 to 10, 1 to 5, or 1 to 3. The substitution, deletion, insertion, and/or addition of one or several amino acids is a conservative mutation that maintains the function of the protein normal. A representative conservative mutation is a conservative substitution. The conservative substitution is, for example, a mutation in which a substitution takes place mutually among Phe, Trp, and Tyr when the substitution site is an aromatic amino acid, among Leu, Ile, and Val when the substitution site is a hydrophobic amino acid, between Gln and Asn when the substitution site is a polar amino acid, among Lys, Arg, and His when the substitution site is a basic amino acid, between Asp and Glu when the substitution site is an acidic amino acid, and between Ser and Thr when the substitution site is an amino acid having a hydroxyl group. Specific examples of the substitution regarded as a conservative substitution include a substitution from Ala to Ser or Thr, a substitution from Arg to Gln, His or Lys, a substitution from Asn to Glu, Gln, Lys, His, or Asp, a substitution from Asp to Asn, Glu, or Gln, a substitution from Cys to Ser or Ala, a substitution from Gln to Asn, Glu, Lys, His, Asp, or Arg, a substitution from Glu to Gly, Asn, Gln, Lys, or Asp, a substitution from Gly to Pro, a substitution from His to Asn, Lys, Gln, Arg, or Tyr, a substitution from Ile to Leu, Met, Val, or Phe, a substitution from Leu to Ile, Met, Val, or Phe, a substitution from Lys to Asn, Glu, Gln, His, or Arg, a substitution from Met to Ile, Leu, Val, or Phe, a substitution from Phe to Trp, Tyr, Met, Ile, or Leu, a substitution from Ser to Thr or Ala, a substitution from Thr to Ser or Ala, a substitution from Trp to Phe or Tyr, a substitution from Tyr to His, Phe, or Trp, and a substitution from Val to Met, Ile, or Leu.

Further, the variant may be, for example, a recombinant protein that contains an amino acid sequence having an 80% or more, 90% or more, 95% or more, 96% or more, 97% or more, 98% or more, or 99% or more identity with an amino acid sequence (for example, the amino acid sequence represented by SEQ ID NO: 2 or 4) of the recombinant first protein as described above, and has factor C activity.

Further, the variant may be, for example, a recombinant protein that is encoded by a DNA containing a base sequence having an 80% or more, 90% or more, 95% or more, 96% or more, 97% or more, 98% or more, or 99% or more identity with a base sequence (for example, the base sequence represented by SEQ ID NO: 1 or 3) encoding the amino acid sequence of the recombinant first protein as described above, and has factor C activity.

Further, the variant may be, for example, a recombinant protein that is encoded by a DNA hybridizing to a DNA composed of a complementary sequence to a base sequence (for example, the base sequence represented by SEQ ID NO: 1 or 3) encoding the amino acid sequence of the recombinant first protein as described above under stringent conditions, and has factor C activity. The term "stringent conditions" refers to conditions under which a so-called specific hybrid is formed and no nonspecific hybrid is formed by a general hybridization operation described in Molecular Cloning: A Laboratory Manual 2nd ed. (1989) Cold Spring Harbor Laboratory edited by T. Maniatis et al., or the like. The term "stringent conditions" specifically refers to conditions in which a sodium salt concentration is from 15 to 750 mM, preferably from 50 to 750 mM, more preferably from 300 to 750 mM, the temperature is from 25 to 70°C, preferably from 50 to 70°C, more preferably from 55 to 65°C, and a formamide concentration is from 0 to 50%, preferably from 20 to 50%, more preferably from 35 to 45%. Further, in the case of stringent conditions, washing conditions for a filter after hybridization are such that a sodium salt concentration is generally from 15 to 600 mM, preferably from 50 to 600 mM, more preferably from 300 to 600 mM, and the temperature is from 50 to 70°C, preferably from 55 to 70°C, more preferably from 60 to 65°C.

As the variant, specifically, the above-mentioned recombinant proteins (B) to (D) are preferred. The term "95% or more" in the above (B) and (C) is preferably 96% or more, more preferably 97% or more, more preferably 98% or more, and more preferably 99% or more.

Further, to the recombinant first protein, an arbitrary peptide such as a His tag or a V tag, or the like may be added as long as it has factor C activity.

Further, the term "recombinant protein" in this application document refers to a protein obtained by artificially introducing a gene encoding a protein into a host cell other than a horseshoe crab and expressing the protein. Therefore, the protein acquired from a natural horseshoe crab itself does not correspond to the "recombinant protein" in this application document.

### (2) Method for Producing Recombinant First Protein

The full-length amino acid sequence of the recombinant first protein has been disclosed for the first time herein, and therefore, the recombinant first protein can be produced by a genetic engineering technique using, for example, a DNA encoding this sequence. A method for producing the recombinant first protein by a genetic engineering technique is not particularly limited, and for example, a general method for producing a protein by a genetic engineering technique can be adopted. The recombinant first protein can be produced by, for example, utilizing a heterologous expression system or a cell-free protein synthesis system.

For example, the recombinant first protein can be produced by artificially introducing the below-mentioned first cDNA into a host cell other than a horseshoe crab and expressing the protein from this cDNA. Also described herein is a method for producing the recombinant first protein.

When artificially introducing a DNA into a host cell, a DNA construct (such as a vector) into which the DNA has been artificially integrated may be used. As such an artificial DNA construct, for example, a DNA construct into which the first cDNA has been integrated can be exemplified. Such an artificial DNA construct will be described later.

The host cell other than a horseshoe crab is not particularly limited as long as it can functionally express the protein from the first cDNA. Examples of the host cell other than a horseshoe crab include mammalian cells and insect cells. Examples of mammals include rodents and primates. Examples of the rodents include Chinese hamsters, hamsters, mice, rats, and guinea pigs. Examples of the Chinese hamster cells include a Chinese hamster ovary-derived cell line (CHO) . Examples of the CHO include CHO DG44, CHO S, and CHO K1. The primates are not particularly limited, however, examples thereof include humans, monkeys, and chimpanzees. Examples of the human cells include a human embryonic kidney cell-derived cell line (HEK) . Examples of the HEK include HEK 293.

The phrase "functionally expressed" refers to that the expressed recombinant first protein shows the factor C activity. Whether a protein shows the factor C activity can be confirmed by the method described in the below-mentioned Example . Further, whether the expressed protein contains a target amino acid sequence of any of the above-mentioned structures (A) to (D) or the like can be confirmed by analyzing the amino acid sequence of the expressed protein and comparing this with the target amino acid sequence.

A technique for artificially introducing the first cDNA into a host cell is also not particularly limited as long as the cDNA is in a state of being expressibly retained in the host cell. By growing the host cell into which the first cDNA has been artificially introduced, the recombinant first protein can be expressed.

The growth of the transformed host cell can be carried out by, for example, culturing the cell. The culturing conditions at this time are also not particularly limited as long as the cell can be grown or proliferated, and conditions generally used for culturing the cell can be used by being appropriately modified as needed. For example, when the host cell is a mammalian cell, a culture medium generally used for culturing the mammalian cell can be used. As such a culture medium, for example, RPMI-1640 medium (Sigma Aldrich Co. LLC.), DMEM medium (Sigma Aldrich Co. LLC.), ExpiCHO^{™} Expression Medium (Thermo Fisher Scientific), or the like can be used. The culturing can be carried out by, for example, static culture, suspension culture, or the like at 36°C to 38°C while supplying 5% to 8% CO₂. A kit dedicated to protein expression may be used.

The expressed recombinant first protein is collected as a solution fraction containing this and can be utilized in various applications described below.

The solution fraction containing the recombinant first protein is a culture solution, or a culture supernatant. The recombinant first protein may be used by being purified to a desired extent or may be used as such without purification.

The purification can be carried out by a known method used for purifying a protein. Examples of such a method include ammonium sulfate precipitation, gel filtration chromatography, ion exchange chromatography, hydrophobic interaction chromatography, and hydroxyapatite chromatography.

As described herein, it has become possible for the first time to artificially and functionally express the recombinant first protein. The thus produced recombinant first protein can be used for, for example, the application described below.

### <2> Recombinant Second Protein and Production Method Therefor

The recombinant second protein is a recombinant protein having factor B activity.

The recombinant second protein is specifically a recombinant protein which is either of the following:
(I) a recombinant protein that contains an amino acid sequence of the protein of Limulus polyphemus having factor B activity; and
(II) a recombinant protein that is a variant of the above (I) and has factor B activity.

Examples of the amino acid sequence of the protein of Limulus polyphemus having factor B activity include the amino acid sequence represented by SEQ ID NO: 6, 8, 10, or 12. Further, examples of a base sequence encoding such an amino acid sequence include the base sequence represented by SEQ ID NO: 5, 7, 9, or 11.

The recombinant second protein may be more specifically a recombinant protein which is any of the following:
(A) a recombinant protein that contains the amino acid sequence represented by SEQ ID NO: 6, 8, 10, or 12;
(B) a recombinant protein that contains an amino acid sequence having a 95% or more identity with the amino acid sequence represented by SEQ ID NO: 6, 8, 10, or 12, and has factor B activity;
(C) a recombinant protein that is encoded by a DNA containing a base sequence having a 95% or more identity with the base sequence represented by SEQ ID NO: 5, 7, 9, or 11, and has factor B activity; and
(D) a recombinant protein that is encoded by a DNA hybridizing to a DNA composed of a complementary sequence to the base sequence represented by SEQ ID NO: 5, 7, 9, or 11 under stringent conditions, and has factor B activity.

The function of this protein is a function of turning into an active form by a protein (active form) having factor C activity such as the first protein having turned into an active form and converting a protein having proclotting enzyme activity such as the below-mentioned third protein into an active form (referred to as "factor B activity" in this application document). The recombinant second protein may be a protein that shows the factor B activity in combination with at least the first protein such as the recombinant first protein and the third protein such as a recombinant third protein.

Whether having the factor B activity can be confirmed by detecting the progress of a cascade reaction when combining a target recombinant protein with a protein (active form) having factor C activity such as the first protein (active form), a protein (inactive form) having proclotting enzyme activity such as the third protein (inactive form), and a substrate for detection. In this system, an endotoxin for converting a protein (inactive form) having factor C activity into an active form may further exist.

To the other explanation, the explanation of the above <1> can apply. That is, for example, "SEQ ID NO. 1 or 3", "SEQ ID NO. 2 or 4", "(recombinant) first protein", "first cDNA", and "factor C" in the above <1> may be replaced by "SEQ ID NO. 5, 7, 9, or 11", "SEQ ID NO. 6, 8, 10, or 12", "(recombinant) second protein", "second cDNA", and "factor B", respectively, and so on.

### <3> Recombinant Third Protein and Production Method Therefor

The recombinant third protein is a recombinant protein having proclotting enzyme activity.

The recombinant third protein is specifically a recombinant protein which is either of the following:
(I) a recombinant protein that contains an amino acid sequence of the protein of Limulus polyphemus having proclotting enzyme activity; and
(II) a recombinant protein that is a variant of the above (I) and has proclotting enzyme activity.

Examples of the amino acid sequence of the protein of Limulus polyphemus having proclotting enzyme activity include the amino acid sequence represented by SEQ ID NO: 14, 16, 18, 20, or 22. Further, examples of a base sequence encoding such an amino acid sequence include the base sequence represented by SEQ ID NO: 13, 15, 17, 19, or 21.

The recombinant third protein may be more specifically a recombinant protein which is any of the following:
(A) a recombinant protein that contains the amino acid sequence represented by SEQ ID NO: 14, 16, 18, 20, or 22;
(B) a recombinant protein that contains an amino acid sequence having a 95% or more identity with the amino acid sequence represented by SEQ ID NO: 14, 16, 18, 20, or 22, and has proclotting enzyme activity;
(C) a recombinant protein that is encoded by a DNA containing a base sequence having a 95% or more identity with the base sequence represented by SEQ ID NO: 13, 15, 17, 19, or 21, and has proclotting enzyme activity; and
(D) a recombinant protein that is encoded by a DNA hybridizing to a DNA composed of a complementary sequence to the base sequence represented by SEQ ID NO: 13, 15, 17, 19, or 21 under stringent conditions, and has proclotting enzyme activity.

The function of this protein is a function of turning into an active form by a protein (active form) having factor B activity such as the second protein having turned into an active form and cleaving the below-mentioned substrate (a protein or a peptide) (referred to as "proclotting enzyme activity" in this application document) . The recombinant third protein may be a protein that shows the proclotting enzyme activity in combination with at least the second protein such as the recombinant second protein.

Whether a protein has the proclotting enzyme activity can be confirmed by detecting the progress of a cascade reaction when combining a target recombinant protein with a protein (active form) having factor B activity such as the second protein (active form) and a substrate for detection. In this system, a protein (active form) having factor C activity for converting a protein (inactive form) having factor B activity into an active form may further exist. Further, in this system, an endotoxin for converting a protein (inactive form) having factor C activity into an active form may further exist.

To the other explanation, the explanation of the above <1> can apply. That is, for example, "SEQ ID NO. 1 or 3", "SEQ ID NO. 2 or 4", "(recombinant) first protein", "first cDNA", and "factor C" in the above <1> may be replaced by "SEQ ID NO. 13, 15, 17, 19, or 21", "SEQ ID NO. 14, 16, 18, 20, or 22", "(recombinant) third protein", "third cDNA", and "proclotting enzyme", respectively, and so on.

### <4> Use of Recombinant First Protein

The recombinant first protein can be used for activating a protein having factor B activity such as the second protein. In other words, described herein is a method for activating a protein having factor B activity including a step of bringing the recombinant first protein into contact with the protein having factor B activity.

Examples of the protein having factor B activity include the second protein.

The second protein is specifically a protein which is either of the following:
(I) a protein that contains an amino acid sequence of the protein of Limulus polyphemus having factor B activity; and
(II) a protein that is a variant of the above (I) and has factor B activity.

The second protein may be more specifically a protein which is any of the following:
(A) a protein that contains the amino acid sequence represented by SEQ ID NO: 6, 8, 10, or 12;
(B) a protein that contains an amino acid sequence having a 95% or more identity with the amino acid sequence represented by SEQ ID NO: 6, 8, 10, or 12, and has factor B activity;
(C) a protein that is encoded by a DNA containing a base sequence having a 95% or more identity with the base sequence represented by SEQ ID NO: 5, 7, 9, or 11, and has factor B activity; and
(D) a protein that is encoded by a DNA hybridizing to a DNA composed of a complementary sequence to the base sequence represented by SEQ ID NO: 5, 7, 9, or 11 under stringent conditions, and has factor B activity.

Further, examples of the protein having factor B activity also include the following proteins:
(III) a protein that contains an amino acid sequence of a protein of a horseshoe crab other than Limulus polyphemus having factor B activity; and
(IV) a protein that is a variant of the above (III) and has factor B activity.

To the explanation of the protein having factor B activity, the explanation of the above <2> can apply.

As described herein, it has been revealed for the first time that the protein (A) of the recombinant first protein has factor C activity. The protein (A) having such a function and the variant thereof are applied to the activation of the protein having factor B activity.

The protein having factor B activity used here may be a protein acquired from a natural substance or a protein produced artificially (for example, the recombinant second protein) as long as it has the above-mentioned structure and function. As described herein, it has become possible for the first time to artificially and functionally express the recombinant second protein and also it has become possible to stably produce the second protein with constant quality without acquiring it from native Limulus polyphemus. Therefore, the protein having factor B activity is preferably the recombinant second protein.

The activation of the protein having factor B activity using the recombinant first protein can be carried out by bringing a molecule of the recombinant first protein (active form) and a molecule of the protein (inactive form) having factor B activity into contact with each other. Incidentally, in order to convert the recombinant first protein into an active form, the recombinant first protein (inactive form) and the protein (inactive form) having factor B activity may be brought into contact with each other in the presence of an endotoxin.

The conditions during this contact are not particularly limited as long as the conditions allow the recombinant first protein (active form) to exhibit the factor C activity so as to be able to activate the protein having factor B activity (when the operation is carried out in the presence of an endotoxin, the conditions further allow the endotoxin to be able to activate the recombinant first protein) . For example, reaction conditions for a known lysate reagent can be adopted.

Whether the protein having factor B activity has been activated can be confirmed by the method described in the below-mentioned Example.

### <5> Use of Recombinant Second Protein

The recombinant second protein can be used for activating a protein having proclotting enzyme activity such as the third protein. In other words, described herein is a method for activating a protein having proclotting enzyme activity including a step of bringing the recombinant second protein into contact with the protein having proclotting enzyme activity.

Examples of the protein having proclotting enzyme activity include the third protein.

The third protein is specifically a protein which is either of the following:
(I) a protein that contains an amino acid sequence of the protein of Limulus polyphemus having proclotting enzyme activity; and
(II) a protein that is a variant of the above (I) and has proclotting enzyme activity.

The third protein may be more specifically a protein which is any of the following:
(A) a protein that contains the amino acid sequence represented by SEQ ID NO: 14, 16, 18, 20, or 22;
(B) a protein that contains an amino acid sequence having a 95% or more identity with the amino acid sequence represented by SEQ ID NO: 14, 16, 18, 20, or 22, and has proclotting enzyme activity;
(C) a protein that is encoded by a DNA containing a base sequence having a 95% or more identity with the base sequence represented by SEQ ID NO: 13, 15, 17, 19, or 21, and has proclotting enzyme activity; and
(D) a protein that is encoded by a DNA hybridizing to a DNA composed of a complementary sequence to the base sequence represented by SEQ ID NO: 13, 15, 17, 19, or 21 under stringent conditions, and has proclotting enzyme activity.

Further, examples of the protein having proclotting enzyme activity also include the following proteins:
(III) a protein that contains an amino acid sequence of a protein of a horseshoe crab other than Limulus polyphemus having proclotting enzyme activity; and
(IV) a protein that is a variant of the above (III) and has proclotting enzyme activity.

To the explanation of the protein having proclotting enzyme activity, the explanation of the above <3> can apply.

The protein having proclotting enzyme activity used here may be a protein acquired from a natural substance or a protein produced artificially (for example, the recombinant third protein) as long as it has the above-mentioned structure and function.

The activation of the protein having proclotting enzyme activity using the recombinant second protein can be carried out by bringing a molecule of the recombinant second protein (active form) and a molecule of the protein (inactive form) having proclotting enzyme activity into contact with each other. Incidentally, in order to convert the recombinant second protein into an active form, the recombinant second protein (inactive form) and the protein (inactive form) having proclotting enzyme activity may be brought into contact with each other in the presence of a protein (active form) having factor C activity. Further, in order to activate this protein having factor C activity, the protein (inactive form) having factor C activity, the recombinant second protein (inactive form), and the protein (inactive form) having proclotting enzyme activity may be brought into contact with one another in the presence of an endotoxin.

The conditions during this contact are not particularly limited as long as the conditions allow the recombinant second protein (active form) to exhibit the factor B activity so as to be able to activate the protein having proclotting enzyme activity (when the operation is carried out in the presence of the protein (active form) having factor C activity, the conditions further allow the protein (active form) having factor C activity to exhibit the factor C activity so as to be able to activate the recombinant second protein, and when the operation is carried out in the presence of an endotoxin, the conditions further allow the endotoxin to be able to activate the protein having factor C activity) . For example, reaction conditions for a known lysate reagent can be adopted.

Whether the third protein has been activated can be confirmed by the method described in the below-mentioned Example.

Examples of the protein having factor C activity include the first protein.

The first protein is specifically a protein which is either of the following:
(I) a protein that contains an amino acid sequence of the protein of Limulus polyphemus having factor C activity; and
(II) a protein that is a variant of the above (I) and has factor C activity.

The first protein may be more specifically a protein which is any of the following:
(A) a protein that contains the amino acid sequence represented by SEQ ID NO: 2 or 4;
(B) a protein that contains an amino acid sequence having a 95% or more identity with the amino acid sequence represented by SEQ ID NO: 2 or 4, and has factor C activity;
(C) a protein that is encoded by a DNA containing a base sequence having a 95% or more identity with the base sequence represented by SEQ ID NO: 1 or 3, and has factor C activity; and
(D) a protein that is encoded by a DNA hybridizing to a DNA composed of a complementary sequence to the base sequence represented by SEQ ID NO: 1 or 3 under stringent conditions, and has factor C activity.

Further, examples of the protein having factor C activity also include the following proteins:
(III) a protein that contains an amino acid sequence of a protein of a horseshoe crab other than Limulus polyphemus having factor C activity; and
(IV) a protein that is a variant of the above (III) and has factor C activity.

To the explanation of the protein having factor C activity, the explanation of the above <1> can apply.

The protein having factor C activity used here may be a protein acquired from a natural substance or a protein produced artificially (for example, the recombinant first protein) as long as it has the above-mentioned structure and function.

To the other explanation, the explanation of the above <4> can apply.

### <6> Use of Recombinant Third Protein

The recombinant third protein can be used for cleaving a substrate. In other words, described herein is a method for cleaving a substrate including a step of bringing the recombinant third protein into contact with the substrate.

This substrate can also be used as the above-mentioned "substrate for detection" and may be a naturally derived substrate or a synthetic substrate. Such a substrate is not particularly limited as long as it is cleaved by the proclotting enzyme activity of the protein (active form) having proclotting enzyme activity such as the third protein (active form).

Examples of the substrate to be cleaved by the proclotting enzyme activity include a protein such as coagulogen and a synthetic substrate represented by a general formula: X-Y-Z (wherein X- is a protecting group bonded to Y through a covalent bond, Y is a peptide residue, and -Z is a signal substance bonded to Y through an amide bond).

The protecting group X is not particularly limited, and a known protecting group for a peptide can be used. Examples of such a protecting group include a t-butoxycarbonyl group and a benzoyl group.

The peptide residue Y is also not particularly limited, and examples thereof include Leu-Gly-Arg (LGR) and Ile-Glu-Gly-Arg (IEGR) (SEQ ID NO: 23).

The signal substance Z is also not particularly limited, and examples thereof include a dye that is detected under visible light and a fluorescent dye. Examples of such a dye include pNA (p-nitroaniline), MCA (7-methoxycoumarin-4-acetic acid), DNP (2,4-dinitroaniline), and a dansyl-type dye.

Among these, Boc-Leu-Gly-Arg-pNA (t-butoxycarbonyl-leucyl-glycyl-arginyl-p-nitroaniline, Boc-LGR-pNA) is preferred.

Whether the substrate has been cleaved by the proclotting enzyme activity can be detected by, for example, detecting "gelation" when coagulogen is used as the substrate. The gelation can be detected by detecting a decrease in fluidity by visual observation or the like.

Further, for example, when a synthetic substrate as described above is used, the signal Z is released by cleaving the amide bond between Y and Z and a signal such as a developed color or fluorescence is emitted, and therefore, by detecting this signal, whether the substrate has been cleaved by the proclotting enzyme activity can be detected. The detection of the signal may be carried out by a technique according to the type of the signal. For example, the signal of pNA can be detected by an absorbance (405 nm).

The cleavage of the substrate using the recombinant third protein can be carried out by bringing a molecule of the recombinant third protein (active form) and a molecule of the substrate into contact with each other. Incidentally, in order to convert the recombinant third protein into an active form, the recombinant third protein (inactive form) and the substrate may be brought into contact with each other in the presence of the protein (active form) having factor B activity. Further, in order to convert this protein having factor B activity into an active form, the protein (inactive form) having factor B activity, the recombinant third protein (inactive form), and the substrate may be brought into contact with one another in the presence of the protein (active form) having factor C activity. Further, in order to convert this protein having factor C activity into an active form, the protein (inactive form) having factor C activity, the protein (inactive form) having factor B activity, the recombinant third protein (inactive form), and the substrate may be brought into contact with one another in the presence of an endotoxin.

The conditions during this contact are not particularly limited as long as the conditions allow the recombinant third protein (active form) to exhibit the proclotting enzyme activity so as to be able to cleave the substrate (when the operation is carried out in the presence of the protein (active form) having factor B activity, the conditions further allow the protein (active form) having factor B activity to exhibit the factor B activity so as to be able to activate the recombinant third protein; when the operation is carried out in the presence of the protein (active form) having factor C activity, the conditions further allow the protein (active form) having factor C activity to exhibit the factor C activity so as to be able to activate the second protein; and when the operation is carried out in the presence of an endotoxin, the conditions further allow the endotoxin to be able to activate the protein having factor C activity) . For example, reaction conditions for a known lysate reagent can be adopted.

To the other explanation, the explanation of the above <4> can apply.

### <7> Method for Detecting Endotoxin

The method for detecting an endotoxin described herein is a method for detecting an endotoxin in a specimen including a step of bringing the recombinant first protein into contact with the specimen.

This method may further include a step of bringing the recombinant first protein having contacted with the specimen into contact with a protein having factor B activity such as the second protein. This protein having factor B activity may be the recombinant second protein.

Further, this method may further include a step of bringing the protein having factor B activity having contacted with the "recombinant first protein having contacted with the specimen" into contact with a protein having proclotting enzyme activity such as the third protein. This protein having proclotting enzyme activity may be the recombinant third protein.

The detection of an endotoxin in a specimen can be carried out by detecting the activation of the recombinant first protein having contacted with the specimen. Whether the recombinant first protein has been activated can be confirmed by the method described in the below-mentioned Example . Further, the activation of the recombinant first protein can also be directly detected by utilizing a substrate (substrate for detection). That is, by utilizing a substrate that emits a signal by being cleaved by the factor C activity of the activated recombinant first protein as the substrate for detection, the activation of the recombinant first protein can be directly detected. Therefore, this method may further include a step of bringing the "recombinant first protein having contacted with the specimen" into contact with the substrate for detection.

When the method further includes the step of bringing into contact with the protein having factor B activity, the detection can be carried out by detecting the activation of the protein having factor B activity by the method described in the above <4>. Further, the activation of the protein having factor B activity can also be directly detected by utilizing a substrate (substrate for detection). That is, by utilizing a substrate that emits a signal by being cleaved by the factor B activity of the activated protein having factor B activity as the substrate for detection, the activation of the protein having factor B activity can be directly detected. Therefore, this method may further include a step of bringing the "protein having factor B activity having contacted with the recombinant first protein" into contact with the substrate for detection.

When the method further includes the step of bringing into contact with the protein having proclotting enzyme activity, the detection can be carried out by detecting the activation of the protein having proclotting enzyme activity by the method described in the above <5> (the cleavage of the substrate by the method described in the above <6>) . Therefore, this method may further include a step of bringing the "protein having proclotting enzyme activity having contacted with the protein having factor B activity" into contact with the substrate (substrate for detection) . Examples of the substrate for detection capable of detecting the activation of the protein having proclotting enzyme activity include those exemplified in the above <6>.

These steps may be carried out sequentially or simultaneously. For example, when these steps are carried out simultaneously, the specimen, the recombinant first protein (inactive form), the protein (inactive form) having factor B activity, the protein (inactive form) having proclotting enzyme activity, and the substrate for detection may be brought into contact with one another simultaneously in the same system.

The conditions for the contact of these are the same as the conditions described in the above <4> to <6>. For example, the contact of the recombinant first protein with the specimen is not particularly limited as long as the conditions allow the endotoxin in the specimen to be able to activate the recombinant first protein. As the conditions for the contact, for example, reaction conditions for a known lysate reagent can be adopted. For example, as a pH of a reaction solution, a pH of 5 to 10, preferably 7 to 8.5 can be adopted. As a reaction temperature, a temperature of 10°C to 80°C, preferably 20°C to 50°C, more preferably 30°C to 40°C can be adopted. As the reaction temperature, for example, 37°C is exemplified. A reaction time is also not particularly limited, and may be appropriately set according to various conditions. The reaction time may be, for example, from 5 minutes to 2 hours, preferably from 15 to 90 minutes, more preferably from 30 to 40 minutes.

The specimen is also not particularly limited as long as it is a sample requiring detection of an endotoxin. Examples of the specimen include medical water, pharmaceutical preparations, infusions, blood preparations, medical devices, medical instruments, cosmetics, foods and drinks, environmental samples, biological components, natural proteins, recombinant proteins, nucleic acids, and saccharides. The specimen can be subjected to the detection of an endotoxin by mixing, dispersing, or dissolving the specimen itself or an extract thereof or a washing solution thereof in a reaction system.

The detection of an endotoxin may be qualitative detection or quantitative detection. The quantitative detection can be carried out, for example, as follows: a plurality of reference standards having different endotoxin amounts (concentrations) are used, a relationship between the amount (concentration) of the endotoxin and the detection level according to the type of the substrate (for example, the degree of "gelation" when coagulogen is used as the substrate, and the degree of a signal when a synthetic substrate is used) is associated in advance using, for example, a calibration curve or a relational formula, and the amount (concentration) of the endotoxin is calculated by conversion from the detection level when using the actual specimen.

### <8> Endotoxin Detecting Agent

The endotoxin detecting agent described herein is an endotoxin detecting agent containing the recombinant first protein.

This detecting agent may further contain a protein having factor B activity such as the second protein. This protein having factor B activity may be the recombinant second protein.

This detecting agent may further contain a protein having proclotting enzyme activity such as the third protein. This protein having proclotting enzyme activity may be the recombinant third protein.

These proteins are in the form of being present in a culture solution, or a culture supernatant. These proteins may be used by being purified to a desired extent or may be used as such without purification. For example, when these proteins are contained in a culture supernatant, the culture supernatant may be used while containing the culture medium components.

The respective amounts of the recombinant first protein, the protein having factor B activity, and the protein having proclotting enzyme activity in this detecting agent can be appropriately set according to the activity or the like of each protein, and, for example, as the final concentration of each protein in the reaction solution in a state of being mixed with the specimen, 15 to 30 µg/mL can be exemplified.

This detecting agent may further contain a substrate (substrate for detection).

This detecting agent can be produced by incorporating these materials as the constituent components. This detecting agent may further contain an additive such as an excipient, a stabilizing agent, or a buffer other than these. Further, the form of this detecting agent is also not particularly limited, and the agent can be formulated into an arbitrary form such as a solid form (for example, a lyophilized form) or a liquid form.

In this detecting agent, these materials may be collectively put in a one-unit container as the constituent components or may be put in separate containers.

Further, this detecting agent may further contain a buffer solution for dissolution, an endotoxin reference standard, a container for a reaction (for example, a tube or a microplate), or the like. This detecting agent also includes a form of a kit.

This detecting agent can be used in, for example, the method described in the above <7>.

### <9> First cDNA and Utilization Thereof

### (1) First cDNA

The first cDNA is a cDNA which is any of the following:
(A) a cDNA that contains the base sequence represented by SEQ ID NO: 1 or 3;
(B) a cDNA that contains a base sequence having a 95% or more identity with the base sequence represented by SEQ ID NO: 1 or 3, and encodes a protein having factor C activity;
(C) a cDNA that hybridizes to a DNA composed of a complementary sequence to the base sequence represented by SEQ ID NO: 1 or 3 under stringent conditions, and encodes a protein having factor C activity;
(D) a cDNA that encodes a protein containing the amino acid sequence represented by SEQ ID NO: 2 or 4; and
(E) a cDNA that encodes a protein containing an amino acid sequence having a 95% or more identity with the amino acid sequence represented by SEQ ID NO: 2 or 4, and having factor C activity.

This cDNA was acquired for the first time in the world by the method disclosed in Examples of this description, and the full-length sequence structure thereof and the function of a protein encoded by the cDNA were elucidated for the first time. The function is "factor C activity".

Among the first cDNAs, the most preferred is the above-mentioned cDNA (A), but the cDNA may be a variant thereof as long as a protein encoded by the cDNA has factor C activity.

As the variant, specifically, the above-mentioned cDNAs (B) to (E) are preferred. The term "95% or more" in the above (B), (C), and (E) is preferably 96% or more, more preferably 97% or more, more preferably 98% or more, and more preferably 99% or more.

Further, to the meaning of the "stringent conditions" and the other explanation, the explanation of the above <1> can apply.

Further, the first cDNA may be a cDNA in which an arbitrary codon is replaced by a codon equivalent thereto. For example, a cDNA in which an arbitrary codon in the above-mentioned cDNA (A) is replaced by a codon equivalent thereto is included in the above (D).

The full-length base sequence of the first cDNA is disclosed herein, and therefore, by using this sequence information, the first cDNA can be produced by a known DNA synthesis technique or a genetic engineering technique. The first cDNA was acquired for the first time by the method described in the below-mentioned Example, however, since the base sequence is disclosed herein, the first cDNA can also be produced by a method other than the method described in Example .

### (2) DNA Construct Containing First cDNA, Transformant, and Method for Producing Recombinant First Protein using the Same

Described herein is a DNA construct containing the first cDNA. As such an artificial DNA construct, for example, a vector can be exemplified. As the vector, a plasmid, a virus, or another known vector can be appropriately selected according to the purpose such as amplification, maintenance, or introduction of a recombinant DNA, library preparation, cloning, protein translation (protein expression), or the like. By introducing the first cDNA into such a vector or the like, the DNA construct containing the first cDNA can be produced.

Further, described herein is a cell containing the first cDNA. Such a cell may be, for example, a cell that is transformed with such a DNA construct so as to express the recombinant first protein. Further, described herein is a method for producing the recombinant first protein including a step of culturing such a cell.

To the method for transforming a cell (host cell) with the DNA construct and the method for producing the recombinant first protein by culturing the cell, the description of the above <1> (2) can apply.

### <10> Second cDNA

### (1) Second cDNA

The second cDNA is a cDNA which is any of the following:
(A) a cDNA that contains the base sequence represented by SEQ ID NO: 5, 7, 9, or 11;
(B) a cDNA that contains a base sequence having a 95% or more identity with the base sequence represented by SEQ ID NO: 5, 7, 9, or 11, and encodes a protein having factor B activity;
(C) a cDNA that hybridizes to a DNA composed of a complementary sequence to the base sequence represented by SEQ ID NO: 5, 7, 9, or 11 under stringent conditions, and encodes a protein having factor B activity;
(D) a cDNA that encodes a protein containing the amino acid sequence represented by SEQ ID NO: 6, 8, 10, or 12; and
(E) a cDNA that encodes a protein containing an amino acid sequence having a 95% or more identity with the amino acid sequence represented by SEQ ID NO: 6, 8, 10, or 12, and having factor B activity.

To the other explanation, the explanation of the above <9> (1) can apply. That is, for example, "factor C" and "first cDNA" in the above <9> (1) may be replaced by "factor B" and "second cDNA", respectively, and so on.

### (2) DNA Construct Containing Second cDNA, Transformant, and Method for Producing Recombinant Second Protein using the Same

Described herein is a DNA construct containing the second cDNA. Further, described herein is a cell containing the second cDNA such as a cell that is transformed with such a DNA construct so as to express the recombinant second protein. Further, described herein is a method for producing the recombinant second protein including a step of culturing such a cell.

To the other explanation, the explanation of the above <9> (2) can apply.

### <11> Third cDNA

### (1) Third cDNA

The third cDNA is a third cDNA which is any of the following:
(A) a cDNA that contains the base sequence represented by SEQ ID NO: 13, 15, 17, 19, or 21;
(B) a cDNA that contains a base sequence having a 95% or more identity with the base sequence represented by SEQ ID NO: 13, 15, 17, 19, or 21, and encodes a protein having proclotting enzyme activity;
(C) a cDNA that hybridizes to a DNA composed of a complementary sequence to the base sequence represented by SEQ ID NO: 13, 15, 17, 19, or 21 under stringent conditions, and encodes a protein having proclotting enzyme activity;
(D) a cDNA that encodes a protein containing the amino acid sequence represented by SEQ ID NO: 14, 16, 18, 20, or 22;
   and
(E) a cDNA that encodes a protein containing an amino acid sequence having a 95% or more identity with the amino acid sequence represented by SEQ ID NO: 14, 16, 18, 20, or 22, and having proclotting enzyme activity.

To the other explanation, the explanation of the above <9> (1) can apply. That is, for example, "factor C" and "first cDNA" in the above <9> (1) may be replaced by "proclotting enzyme" and "third cDNA", respectively, and so on.

### (2) DNA Construct Containing Third cDNA, Transformant, and Method for Producing Recombinant Third Protein using the Same

Described herein is a DNA construct containing the third cDNA. Further, described herein is a cell containing the third cDNA such as a cell that is transformed with such a DNA construct so as to express the recombinant third protein. Further, described herein is a method for producing the recombinant third protein including a step of culturing such a cell.

To the other explanation, the explanation of the above <9> (2) can apply.

### <12> Method for Producing Endotoxin Detecting Agent

Also described herein is a method for producing an endotoxin detecting agent including a step of artificially expressing a protein using the first cDNA.

This method may further include a step of artificially expressing a protein using the second cDNA. In addition, the method may further include a step of artificially expressing a protein using the third cDNA.

A method for artificially expressing a protein using such a cDNA is also not particularly limited, however, for example, the method described in the above <9> (2), <10> (2), or <11> (2) can be used.

The recombinant first protein is expressed by being artificially expressed using the first cDNA according to such a method. Similarly, the recombinant second protein is expressed by being artificially expressed using the second cDNA, and the recombinant third protein is expressed by being artificially expressed using the third cDNA.

The expressed protein is in the form of being present in a culture solution, or a culture supernatant. These proteins may be used by being purified to a desired extent or may be used as such without purification. For example, when these proteins are contained in a culture supernatant, the culture supernatant may be used while containing the culture medium components.

To the other explanation, the explanation of the above <8> can apply. Therefore, the method for producing an endotoxin detecting agent described herein also includes the method for producing a kit.

Incidentally, either one or both of the second protein (such as the recombinant second protein) and the third protein (such as the recombinant third protein) may be replaced by their equivalents derived from a horseshoe crab other than Limulus polyphemus. The same also apply to the second cDNA and the third cDNA. Examples of the horseshoe crab other than Limulus polyphemus include organisms belonging to the genus Tachypleus such as Tachypleus tridentatus, Tachypleus gigas, and Tachypleus rotundicauda.

That is, in place of the second protein (such as the recombinant second protein), factor B (such as recombinant factor B) derived from a horseshoe crab (such as Tachypleus tridentatus) other than Limulus polyphemus, in place of the third protein (such as the recombinant third protein), a proclotting enzyme (such as a recombinant proclotting enzyme) derived from the same organism, in place of the second cDNA, a factor B gene (such as a cDNA) derived from the same organism, and in place of the third cDNA, a proclotting enzyme gene (such as a cDNA) derived from the same organism may be used, respectively.

These proteins and genes may all be those containing an amino acid sequence or a base sequence actually found in a horseshoe crab other than Limulus polyphemus, or may be variants thereof. To the variants, the explanation of the variant in the above <1> to <11> can apply. Further, these proteins and genes may be or may not be derived from the same horseshoe crab other than Limulus polyphemus.

Then, as apparent also from the above-mentioned description, in the method for producing an endotoxin detecting agent described herein, a case in which each of the first cDNA, the second cDNA, and the third cDNA (with respect to the second cDNA and the third cDNA, either one or both may be replaced by a gene derived from a horseshoe crab (such as Tachypleus tridentatus) other than Limulus polyphemus) is artificially introduced into a host cell other than a horseshoe crab, the respective recombinant proteins (three types in total) are expressed from these respective cDNAs, and then, the expressed respective recombinant proteins are incorporated as the constituent components as described in the above <8> is also included.

### Examples

Hereinafter, the present invention will be specifically described by way of Examples, however, these are merely examples of the present invention, and the scope of the present invention is not limited thereto.

### (1) Synthesis of Primers

In order to attempt cloning of a protein having factor C activity in Limulus polyphemus, the following respective primers were synthesized.

### <Primers Used in Cloning of Protein Having Factor C Activity>

SK15-dT20: CTGCAGGAATTCGATTTTTTTTTTTTTTTTTTTTT (SEQ ID NO: 24)
SK15-FC-S: ATCGATAAGCTTGATGATCTGGGCTTGTGTGATGA (SEQ ID NO: 25)
SK15-As: CTGCAGGAATTCGAT (SEQ ID NO: 26)
LFC-5race: CTACACCAAGTTCCA (SEQ ID NO: 27)
LFC-1st-S: GTAAACCATGTGACAAACTGGAGGC (SEQ ID NO: 28)
LFC-1st-As: AATAAGGCCTCCATCGATAGAAGTA (SEQ ID NO: 29)
LFC-EcoRI-kozak-S: GGGGAATTCAAGCTTGCCACCATGGTACTAGCGTCGTTC (SEQ ID NO: 30)
LFC-XhoI-stop-As: GGGCTCGAGTCAAATGAACTGCCGAATCCACGATA (SEQ ID NO: 31)

Further, in order to attempt cloning of each of a protein having factor B activity and a protein having proclotting enzyme activity, the following respective primers were synthesized.

### <Primers Used in Cloning of Protein Having Factor B Activity and Protein Having Proclotting Enzyme Activity>

SK15-FB-S: ATCGATAAGCTTGATCACATGCAAGGAAAAGTTCT (SEQ ID NO: 32)
SK15-FB-As: CTGCAGGAATTCGATCACTGTTTAAACAAACTGAA (SEQ ID NO: 33)
SK15-PCE-S: ATCGATAAGCTTGATAGACCAGAGTGGTCTTTCTG (SEQ ID NO: 34)
SK15-As: CTGCAGGAATTCGAT (SEQ ID NO: 26)

### (2) Preparation of Total RNA from Blood Cells of Limulus polyphemus

In the preparation of total RNA, PureLink (registered trademark) RNA Mini Kit and PureLink (registered trademark) DNase kit (Thermo Fisher Scientific) were used, and the basic operation was carried out according to the accompanying instructions. A TRIzol solution was added to 2.23 g of a blood cell pool of Limulus polyphemus, and the cells were homogenized. Thereafter, chloroform was added thereto, followed by centrifugation, whereby an aqueous layer was obtained. After ethanol was added to the obtained aqueous layer, the resulting mixture was allowed to pass through a silica membrane cartridge included in the kit, whereby a nucleic acid component was bound to the silica membrane. To this silica membrane, DNase I included in the kit was added so as to degrade the DNA, and after the cartridge was washed, an elution buffer was added thereto, whereby total RNA (1.3 mg) was collected.

### (3) Acquisition of cDNA (First cDNA) of Protein Having Factor C Activity of Limulus polyphemus

### (3-1) First Step

Reverse transcription was carried out using the total RNA collected in the above (2) as a template and SK15-d20 (SEQ ID NO: 24) as a primer, whereby a cDNA was obtained. This primer was designed so as to selectively bind to the poly (A) sequence of mRNA and further has an addition sequence that can be utilized in a polymerase chain reaction (PCR) on the outside thereof. In this reaction, SuperScript III Reverse Transcriptase (Thermo Fisher Scientific) was used as a reverse transcriptase, and the reaction conditions were set according to the accompanying instructions.

### (3-2) Second Step

PCR was carried out using the obtained cDNA as a template, SK15-FC-S (SEQ ID NO: 25) as a sense primer, SK15-As (SEQ ID NO: 26) as an antisense primer, and Tks Gflex DNA polymerase (Takara Bio Inc.) as a polymerase.

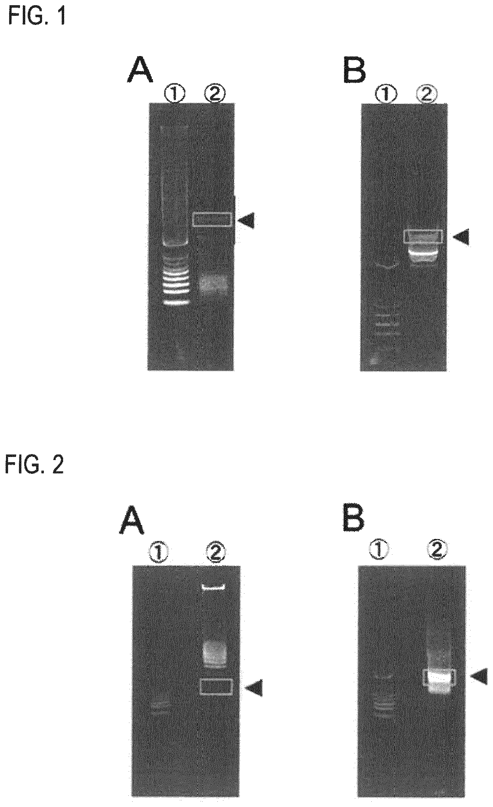

When the PCR product was confirmed by agarose gel electrophoresis, a target DNA (LpFC1) almost could not be detected, and nonspecific DNA amplification was detected (A of FIG. 1). Therefore, the following step was attempted. Incidentally, in A of FIG. 1, the lane "1" is a size marker and the lane "2" is the PCR product. Further, the triangle mark indicates a portion where the presence of LpFC1 is expected.

### (3-3) Third Step

The portion where the presence of LpFC1 was expected was cut out from the gel, and a DNA was extracted and purified. PCR was carried out using the obtained DNA as a template, SK15-FC-S (SEQ ID NO: 25) as a sense primer, SK15-As (SEQ ID NO: 26) as an antisense primer, and Tks Gflex DNA polymerase (Takara Bio Inc.) as a polymerase.

As a result of subjecting the PCR product to agarose gel electrophoresis, surprisingly, the target DNA (LpFC1) was remarkably amplified (B of FIG. 1) . This LpFC1 was cut out from the gel and extracted and purified. By doing this, the acquisition of a partial fragment (LpFC1) of a DNA that may encode the protein having factor C activity of Limulus polyphemus was achieved. Incidentally, in B of FIG. 1, the lane "1" is a size marker and the lane "2" is the PCR product. Further, the triangle mark indicates the position of LpFC1.

### (3-4) Fourth Step

LpFC1 was mixed with pBlueScript II SK (+) treated with EcoRV enzyme and introduced into a vector by recombination using In-Fusion HD Enzyme premix (Takara Bio Inc.). The reaction product was introduced into E. coli DH5α competent cells, and the cells were applied to an ampicillin LB medium plate containing X-Gal and IPTG, and colony selection was carried out by blue white screening.

Thereafter, DNA amplification was carried out by colony PCR for a candidate colony. At this time, M13-20 Primer was used as a sense primer, M13 Reverse Primer was used as an antisense primer, and Tks Gflex DNA polymerase (Takara Bio Inc.) was used as a polymerase. After the obtained PCR product was subjected to agarose gel electrophoresis, a DNA (LpFC1) obtained by cutting out from the gel, extraction, and purification was used as a template, and the sequence thereof was confirmed by sequence PCR. By doing this, the DNA sequence of LpFC1 was identified.

### (3-5) Fifth Step

The 5' end of synthetic DNA LFC-5race (SEQ ID NO: 27) prepared based on the sequence information of LpFC1 was phosphorylated with T4 Polynucleotide Kinase (Takara Bio Inc.) in the presence of ATP. A target DNA (LpFC2) was obtained by reverse transcription using the total RNA as a template and also using the obtained phosphorylated primer and SuperScript III Reverse Transcriptase (Thermo Fisher Scientific) as a reverse transcriptase. By doing this, a partial fragment (LpFC2) of a DNA that may encode an amino acid sequence containing a portion of the protein of Limulus polyphemus having factor C activity was obtained.

LpFC2 was subjected to a high-temperature treatment (95°C, 2 minutes) in the presence of RNase A, followed by ethanol precipitation. Thereafter, the resulting material was treated with T4 RNA Ligase (New England Biolabs), and as a result, a concatenated DNA (LpFC3) in which LpFC2 is linked in series was obtained. By doing this, a partial fragment (LpFC3) of a DNA that may encode an amino acid sequence containing a portion of the protein of Limulus polyphemus having factor C activity was obtained.

PCR was carried out using LpFC3 as a template, LFC-1st-S (SEQ ID NO: 28) as a sense primer, LFC-1st-As (SEQ ID NO: 29) as an antisense primer, and Tks Gflex DNA polymerase (Takara Bio Inc.), whereby a target DNA (LpFC4) was amplified.

When the PCR product was confirmed by agarose gel electrophoresis, the target DNA (LpFC4) almost could not be detected, and nonspecific DNA amplification was detected (A of FIG. 2). Therefore, the following step was attempted. Incidentally, in A of FIG. 2, the lane "1" is a size marker and the lane "2" is the PCR product. Further, the triangle mark indicates the position of LpFC4.

### (3-6) Sixth Step

The portion where the presence of LpFC4 was expected was cut out from the gel, and a DNA was extracted and purified. PCR was carried out using the obtained DNA as a template, LFC-1st-S (SEQ ID NO: 28) as a sense primer, LFC-1st-As (SEQ ID NO: 29) as an antisense primer, and Tks Gflex DNA polymerase (Takara Bio Inc.) as a polymerase.

As a result of subjecting the PCR product to agarose gel electrophoresis, surprisingly, the target DNA (LpFC4) was remarkably amplified and detected at a very high concentration (B of FIG. 2) . This LpFC4 was cut out from the gel and extracted and purified. By doing this, the acquisition of a partial fragment (LpFC4) of a DNA that may encode an amino acid sequence containing a portion of the protein having factor C activity of Limulus polyphemus was achieved. Incidentally, in B of FIG. 2, the lane "1" is a size marker and the lane "2" is the PCR product. Further, the triangle mark indicates a portion where the presence of LpFC4 is expected.

The sequence of LpFC4 was analyzed by sequence PCR using LpFC4 as a template. By doing this, the DNA sequence on the 5' side of the target protein was identified.

### (3-7) Seventh Step

PCR was carried out using the cDNA obtained in the above (3-1) as a template, LFC-EcoRI-kozak-S (SEQ ID NO: 30) as a sense primer, LFC-XhoI-stop-As (SEQ ID NO: 31) as an antisense primer, and Tks Gflex DNA polymerase (Takara Bio Inc.) as a polymerase.

The PCR product was purified by phenol-chloroform extraction and ethanol precipitation, and then, treated with EcoRI enzyme and XhoI enzyme. A restriction enzyme-treated product was subjected to agarose gel electrophoresis and cut out from the gel, and a DNA was extracted and purified, whereby a target DNA (LpFC5) was obtained.

LpFC5 was mixed with an expression vector pCA7 (Takeda et al., 2005, J virol 79: 14346-14354) treated with EcoRI enzyme and XhoI enzyme, and a ligation reaction was carried out using Ligation Mix (Takara Bio Inc.). The reaction product was introduced into E. coli DH5α competent cells, and the cells were applied to an ampicillin-containing LB medium plate. From the obtained colonies, selection was carried out by colony PCR, and the selected colony was inoculated into an ampicillin-containing LB medium.

After culturing, plasmid purification was carried out by NucleoSpin (registered trademark) Plasmid Easy Pure (MACHEREY-NAGEL GmbH & Co. KG) . The obtained two types of clones of plasmids were used as templates, and the base sequence of each clone was analyzed by sequence PCR.

By doing this, the elucidation of the full-length sequences (SEQ ID NO: 1 and SEQ ID NO: 3) of cDNAs encoding a protein that may have the factor C activity of Limulus polyphemus, and the full-length sequences (SEQ ID NO: 2 and SEQ ID NO: 4) of amino acids of the protein was achieved for the first time. SEQ ID NOS: 1 and 3 (SEQ ID NOS: 2 and 4) have a variant relationship and are considered to be based on polymorphism between individuals. The expression and the analysis of activity of this protein will be described later.

### (4) Acquisition of cDNA (Second cDNA) of Protein Having Factor B Activity of Limulus polyphemus

PCR was carried out using the cDNA obtained in the above (3-1) as a template, SK15-FB-S (SEQ ID NO: 32) as a sense primer, SK15-FB-As (SEQ ID NO: 33) as an antisense primer, and Tks Gflex DNA polymerase (Takara Bio Inc.) as a polymerase. The amplified fragments were subjected to cloning according to the same procedure as in the above (3-4), and with respect to the obtained four types of clones, the base sequences thereof were analyzed.

By doing this, the full-length sequences (SEQ ID NO: 5, SEQ ID NO: 7, SEQ ID NO: 9, and SEQ ID NO: 11) of cDNAs encoding a protein that may have the factor B activity of Limulus polyphemus, and the full-length sequences (SEQ ID NO: 6, SEQ ID NO: 8, SEQ ID NO: 10, and SEQ ID NO: 12) of amino acids of the protein were elucidated. SEQ ID NOS: 5, 7, 9, and 11 (SEQ ID NOS: 6, 8, 10, and 12) have a variant relationship and are considered to be based on polymorphism among individuals. The expression and the analysis of activity of this protein will be described later.

### (5) Acquisition of cDNA (Third cDNA) of Protein Having Proclotting Enzyme Activity of Limulus polyphemus

PCR was carried out using the cDNA obtained in the above (3-1) as a template, SK15-PCE-S (SEQ ID NO: 34) as a sense primer, SK15-As (SEQ ID NO: 26) as an antisense primer, and Tks Gflex DNA polymerase (Takara Bio Inc.) as a polymerase. The amplified fragments were subjected to cloning according to the same procedure as in the above (3-4), and with respect to the obtained five types of clones, the base sequences thereof were analyzed.

By doing this, the full-length sequences (SEQ ID NO: 13, SEQ ID NO: 15, SEQ ID NO: 17, SEQ ID NO: 19, and SEQ ID NO: 21) of cDNAs encoding a protein that may have the proclotting enzyme activity of Limulus polyphemus, and the full-length sequences (SEQ ID NO: 14, SEQ ID NO: 16, SEQ ID NO: 18, SEQ ID NO: 20, and SEQ ID NO: 22) of amino acids of the protein were elucidated. SEQ ID NOS: 13, 15, 17, 19, and 21 (SEQ ID NOS: 14, 16, 18, 20, and 22) have a variant relationship and are considered to be based on polymorphism among individuals. The expression and the analysis of activity of this protein will be described later.

### (6) Expression of Recombinant Protein

The cDNA (SEQ ID NO: 13) obtained in the above (5) was ligated to a vector pCA7 for protein expression in mammalian cells, whereby an expression plasmid was obtained. The cDNA (SEQ ID NO: 5) obtained in the above (4) was ligated to the vector pCA7 for protein expression according to a literature (Kobayashi et al., 2015, J Biol Chem, 290: 19379-19386), whereby an expression plasmid was obtained. By using these expression plasmids and the expression plasmid (in which the cDNA of SEQ ID NO: 1 was integrated) obtained in the above (3-7), and ExpiCHO^{™} Expression System (Thermo Fisher Scientific), recombinant proteins thereof were obtained as culture supernatant fractions.

The transfection of the expression plasmids into cells and collection of the recombinant proteins were carried out according to the accompanying instructions.

By doing this, the expression and acquisition of the protein (SEQ ID NO: 2) that may have the factor C activity of Limulus polyphemus, the protein (SEQ ID NO: 6) that may have the factor B activity thereof, and the protein (SEQ ID NO: 14) that may have the proclotting enzyme activity thereof were achieved for the first time. The analysis of the activity of these proteins will be described below.

### (7) Measurement of Activity

In the presence of 50 mM Tris buffer solution (pH 8.0) and a synthetic substrate (Boc-LGR-pNA), with respect to various combinations of the three types of recombinant proteins obtained in the above (6), activation of the three types of recombinant proteins depending on an endotoxin was examined using a United States Pharmacopeia reference standard endotoxin (USP-RSE, Seikagaku Corporation) (0 EU/mL and 0.05 EU/ml, EU is the endotoxin unit) as a specimen. This reaction was carried out at 37°C for 30 minutes.

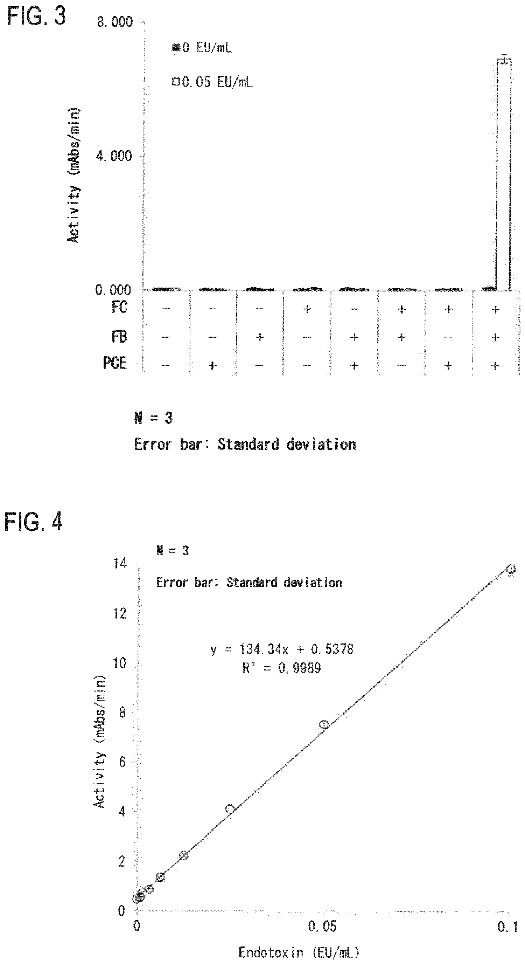

If a cascade reaction proceeds in this assay system, the protein having factor C activity is activated by the endotoxin, the protein having factor B activity is activated by this activated protein having factor C activity, the protein having proclotting enzyme activity is activated by this activated protein having factor B activity, and the synthetic substrate Boc-LGR-pNA is cleaved by this activated protein having proclotting enzyme activity, whereby pNA is released. The release of pNA was detected by measuring a change in the absorbance (A405 nm). As a result, when all the three types of recombinant proteins were included, cleavage of the synthetic substrate was observed (FIG. 3). Incidentally, in FIG. 3, FC denotes the protein expressed from the cDNA obtained in the above (3-7), FB denotes the protein expressed from the cDNA obtained in the above (4), and PCE denotes the protein expressed from the cDNA obtained in the above (5). The final concentrations of the proteins in the reaction solution (100 µL) in a state of being mixed with the endotoxin specimen were as follows: FC: 22.8 µg/mL; FB: 20.0 µg/mL; and PCE: 23.5 µg/mL. Each assay was carried out by setting N=3, and a standard deviation was also shown in the drawing as an error bar.

From this, it was shown for the first time that the expressed protein that may have the factor C activity, the expressed protein that may have the factor B activity, and the expressed protein that may have the proclotting enzyme activity have the factor C activity, the factor B activity, and the proclotting enzyme activity, respectively, and the cascade reaction is caused by contact with an endotoxin. Further, it was shown for the first time that by utilizing these recombinant proteins, an endotoxin can be detected.

Further, when the concentration dependence of an endotoxin was examined under the conditions and a calibration curve was created, it showed favorable linearity within a concentration range from 0.001 to 0.1 EU/mL (FIG. 4) . The assay at each endotoxin concentration was carried out by setting N=3, and a standard deviation was also shown in the drawing as an error bar.

From the above results, it was shown for the first time that by using the above-mentioned respective three types of recombinant proteins, an endotoxin can also be quantitatively detected with high sensitivity and high accuracy.

### (8) Comparison of Activity with Recombinant Factor C Derived from Genus Tachypleus

The activity was compared between recombinant factor C derived from Limulus polyphemus (hereinafter referred to as "LFC") and recombinant factor C derived from the genus Tachypleus (hereinafter referred to as "TFC").

LFC was obtained as a culture supernatant fraction by expressing LFC from the cDNA of SEQ ID NO: 1 in the same manner as in the above (6) . TFC was obtained as a culture supernatant fraction by expressing TFC from a factor C gene of Tachypleus tridentatus (SEQ ID NO: 1 in WO 2014/092079) in the same manner as in the above (6) by the method described in Example 1. (2) in the same literature . Here, as a vector for protein expression, pCI-neo (Promega) was used for both.

With respect to LFC and TFC to be subjected to a test, Western blot was carried out using a factor C specific monoclonal antibody (2C12; Yoshiki Miura, et al., J. Biochem. 112: 476-481 (1992)). As a result of applying an equal volume of samples (3 µL each) and optically measuring the intensities of the bands, the intensity of LFC was 18036 and the intensity of TFC was 24516. That is, the relative amount (TFC/LFC) of the recombinant factor C in the equal volume of the samples was 1.4.

Subsequently, the activity of each recombinant factor C in a cascade reaction system (a system containing factor C, factor B, and a proclotting enzyme) was compared. As both the factor B and the proclotting enzyme, recombinant proteins derived from the genus Tachypleus were used. The recombinant proteins obtained as culture supernatant fractions by expressing the former from a factor B gene of Tachypleus tridentatus (SEQ ID NO: 5 in WO 2014/092079) and by expressing the latter from a proclotting enzyme gene of the same organism (SEQ ID NO: 7 in the same literature) using the method described in the same literature (Example 2. Preparation of Recombinant Factor B and Recombinant Proclotting Enzyme) were used.

By using each sample (the same volume for each) of the recombinant factor C (LFC or TFC), and the recombinant factor B (TFB) derived from Tachypleus tridentatus described above and the recombinant proclotting enzyme (TPCE) derived from the same organism, the activity was measured by the method described in the above (7). The conditions were set the same except for the recombinant factor C to be used.

The changes in the absorbance (mAbs/min) at the respective endotoxin concentrations of 0 EU/mL (blank) and 0.05 EU/mL in the respective cascade reaction systems were 0.53 and 14.27 in the system of LFC, and 0.51 and 9.15 in the system of TFC. That is, in this system, LFC showed the activity about 1.6 times (about 2.2 times when considering the difference in the protein amount) higher than TFC.

As described herein, all full-length recombinant proteins involved in the clotting mechanism of Limulus polyphemus, cDNAs encoding the same, and applications thereof can be provided. The present invention is particularly useful for detecting an endotoxin.

### [Description of Sequence Listing]

SEQ ID NO: 1: base sequence of first cDNA
SEQ ID NO: 2: amino acid sequence of first protein
SEQ ID NO: 3: base sequence of first cDNA variant 1
SEQ ID NO: 4: amino acid sequence of first protein variant 1
SEQ ID NO: 5: base sequence of second cDNA
SEQ ID NO: 6: amino acid sequence of second protein
SEQ ID NO: 7: base sequence of second cDNA variant 1
SEQ ID NO: 8: amino acid sequence of second protein variant 1
SEQ ID NO: 9: base sequence of second cDNA variant 2
SEQ ID NO: 10: amino acid sequence of second protein variant 2
SEQ ID NO: 11: base sequence of second cDNA-variant 3
SEQ ID NO: 12: amino acid sequence of second protein variant 3
SEQ ID NO: 13: base sequence of third cDNA
SEQ ID NO: 14: amino acid sequence of third protein
SEQ ID NO: 15: base sequence of third cDNA variant 1
SEQ ID NO: 16: amino acid sequence of third protein variant 1
SEQ ID NO: 17: base sequence of third cDNA variant 2
SEQ ID NO: 18: amino acid sequence of third protein variant 2
SEQ ID NO: 19: base sequence of third cDNA variant 3
SEQ ID NO: 20: amino acid sequence of third protein variant 3
SEQ ID NO: 21: base sequence of third cDNA variant 4
SEQ ID NO: 22: amino acid sequence of third protein variant 4
SEQ ID NO: 23: synthetic substrate peptide IEGR
SEQ ID NO: 24 to 34: primers

## Claims

1. A method for producing an endotoxin detecting agent, comprising a step of artificially expressing a protein using a cDNA, and collecting the expressed recombinant protein as a solution fraction containing the expressed recombinant protein,
wherein the cDNA encodes any one of the following:
(A) a recombinant protein expressed as containing the amino acid sequence represented by SEQ ID NO: 2 or 4;
(B) a recombinant protein expressed as containing an amino acid sequence having a 95% or more identity with the amino acid sequence represented by SEQ ID NO: 2 or 4, and has factor C activity; and
(C) a recombinant protein that is encoded by a DNA containing a base sequence having a 95% or more identity with the base sequence represented by SEQ ID NO: 1 or 3, and has factor C activity, and
wherein the solution fraction containing the expressed recombinant protein is a culture solution, or a culture supernatant.

2. A method for detecting an endotoxin in a sample requiring detection of endotoxin using the endotoxin detecting agent produced according to the method according to claim 1, comprising a step of bringing the recombinant protein into contact with the sample requiring detection of endotoxin.

## Patentansprüche

1. Verfahren zur Herstellung eines Endotoxin-Nachweismittels, umfassend einen Schritt der künstlichen Expression eines Proteins unter Verwendung einer cDNA und das Sammeln des exprimierten rekombinanten Proteins als eine lösliche Fraktion, die das exprimierte rekombinante Protein enthält,
wobei die cDNA ein jegliches der folgenden kodiert:
(A) ein rekombinantes Protein, das so exprimiert wird, dass es die durch SEQ ID NO: 2 oder 4 dargestellte Aminosäuresequenz enthält;
(B) ein rekombinantes Protein, das so exprimiert wird, dass es eine Aminosäuresequenz enthält, die 95% oder mehr Identität zu der durch SEQ ID NO: 2 oder 4 dargestellten Aminosäuresequenz aufweist, und das Faktor-C-Aktivität aufweist; und
(C) ein rekombinantes Protein, das von einer DNA kodiert wird, die eine Basensequenz enthält, die 95 % oder mehr Identität zu der durch SEQ ID NO: 1 oder 3 dargestellten Basensequenz aufweist, und das Faktor-C-Aktivität aufweist, und
wobei die lösliche Fraktion, die das exprimierte rekombinante Protein enthält, eine Kulturlösung oder ein Kulturüberstand ist.

2. Verfahren zum Nachweis eines Endotoxins in einer Probe, die den Nachweis von Endotoxin erfordert, unter Verwendung des Endotoxin-Nachweismittels, das gemäß dem Verfahren nach Anspruch 1 hergestellt wurde, umfassend einen Schritt des Inkontaktbringens des rekombinanten Proteins mit der Probe, die den Nachweis von Endotoxin erfordert.

## Revendications

1. Procédé de production d'un agent de détection d'endotoxine, comprenant une étape consistant à exprimer artificiellement une protéine en utilisant un ADNc, et recueillir la protéine recombinée exprimée en tant que fraction de solution contenant la protéine recombinée exprimée,
dans lequel l'ADNc code pour l'une quelconque des protéines suivantes :
(A) une protéine recombinée exprimée comme contenant la séquence d'acides aminés représentée par SEQ ID N° : 2 ou 4 ;
(B) une protéine recombinée exprimée comme contenant une séquence d'acides aminés ayant une identité de 95 % ou plus avec la séquence d'acides aminés représentée par SEQ ID N° : 2 ou 4, et a une activité de facteur C ; et
(C) une protéine recombinée qui est codée par un ADN contenant une séquence de bases ayant une identité de 95 % ou plus avec la séquence de bases représentée par SEQ ID N° : 1 ou 3, et a une activité de facteur C, et
dans lequel la fraction de solution contenant la protéine recombinée exprimée est une solution de culture, ou un surnageant de culture.

2. Procédé de détection d'une endotoxine dans un échantillon nécessitant la détection d'endotoxine utilisant l'agent de détection d'endotoxine produit conformément au procédé selon la revendication 1, comprenant une étape consistant à mettre la protéine recombinée en contact avec l'échantillon nécessitant la détection d'endotoxine.
